# EUROPEAN PATENT SPECIFICATION

(11) **EP 3 454 741 B1**
(45) Date of publication and mention of the grant of the patent: **20.10.2021**
(21) Application number: 17795197.7
(22) Date of filing: 12.05.2017
(51) Int. Cl.: A61B 5/097

(54) **A MIND-ALTERING SUBSTANCE TESTING SYSTEM**
TESTSYSTEM FÜR BEWUSSTSEINSVERÄNDERNDE SUBSTANZEN
SYSTÈME DE TEST DE SUBSTANCE PSYCHOTROPE

(30) Priority: 13.05.2016 AU 2016901780
(43) Date of publication of application: 20.03.2019
(73) Proprietor: Alcolizer Pty Ltd, Brisbane, Queensland 4000 (AU)
(72) Inventor: HUNT, Roger Alan, Brisbane Queensland 4000 (AU); BROWN, James John, Brisbane Queensland 4000 (AU)
(74) Representative: Dehns
(86) International application number: PCT/AU2017/050438
(87) International publication number: WO 2017/193176

(56) References cited:
- WO-A1-2011/143693
- WO-A1-2011/143693
- WO-A1-2014/176556
- WO-A2-2009/083964
- US-A1- 2011 050 407
- US-A1- 2012 302 907
- US-A1- 2015 226 727

## Description

### TECHNICAL FIELD

The present invention generally relates to a mind-altering substance testing system used in testing for the presence of mind-altering substances, such as alcohol or drugs.

### BACKGROUND

The reference to any prior art in this specification is not, and should not be taken as an acknowledgement or any form of suggestion that the prior art forms part of the common general knowledge.

Mind-altering substances such as alcohol or drugs may impair human performance.

In Australia, drivers may be stopped along any road by a police officer for a "random breath test", commonly referred to as an "RBT". For an operation involving a large number of police (typically 10-20) at a fixed location, the colloquial term "booze bus" is often used. The RBT is conducted using a hand-held RBT unit in which the driver is required to blow (or breath). The RBT unit senses the driver's blood alcohol content (BAC) from the sampled breath, and a maximum BAC of 0.05% is typically enforced. Drivers who are found to have a preliminary BAC reading of greater than 0.05% are required to wait for a predetermined period, after which time they are retested.

In recent times, random drug testing has been performed by police. However, random drug testing is not as straightforward as conducting an alcohol RBT. Random drug testing involves swabbing the driver before analyzing the sample using an analyzer, usually located in the police vehicle. RBT units such as those disclosed in WO2011/143693 relate to hand-held random breath test (RBT) units including a display module for displaying an indication of blood alcohol content (BAC). However, these RBT units do not include features such as releasably fastened sensor cartridges for receiving saliva samples.

The preferred embodiment provides an improved mind-altering substance testing unit.

### SUMMARY OF THE INVENTION

According to one aspect of the present invention, there is provided a mind-altering substance testing unit as defined in claim 1. Additional aspects are defined in the dependent claims.

The unit may further include a swab including the sample and for fastening to the cartridge. The sample is saliva.

The mind-altering substance may be a drug, and the sensor module may include a lateral flow sensor. The cartridge may be disposable. The cartridge may include an electronic memory for storing information. The information may relate to the age of the cartridge or batch parameters of the cartridge. The sensor module may compensate sensing in accordance with the stored information for improved accuracy.

The mind-altering substance may be alcohol and the sensor module may include an electro-chemical fuel cell sensor blood alcohol content (BAC). The sensor module may include a mouthpiece for releasably fastening to the sensor module in place of the sample cartridge.

The unit may be hand-held. The sensor may be wholly enclosed within the sensor module.

According to another aspect of the present disclosure, there is provided a mind-altering substance testing unit including:
a display module for displaying an indication of a drug or alcohol;
a sensor module for releasably fastening to the display module and including sensors for sensing the drug and alcohol; and
a sample cartridge for receiving a sample and for releasably fastening to the sensor module so that the sensor module can sense the drug.

Advantageously, the same testing unit can be used to sense alcohol and the drug avoiding the need for separate testing units.

According to another aspect of the present disclosure, there is provided a mind-altering substance testing unit including:
a sample cartridge for receiving a sample and for releasably fastening to a sensor so that the sensor can sense the mind-altering substance.

According to another aspect of the present disclosure, there is provided a sample cartridge for a mind-altering substance testing unit, the sample cartridge configured to be releasably fastened to a sensor module so that the sensor module can sense the mind-altering substance.

According to another aspect of the present disclosure, there is provided a hand-held random breath test (RBT) unit including:
a display module for displaying an indication of blood alcohol content (BAC); and
a sensor module for releasably fastening to the display module and enclosing a sensor for sensing the BAC.

The sensor module can be released from the display module for calibration, and houses the sensor to impede damage to the sensor or the compromising of its calibration. Preferably, the sensor is completely contained or encapsulated within the sensor module and cannot be accessed with a finger. The sensor may be fluidically sealed in an air-tight or water-tight manner within the sensor module. The sensor module may be disposable, and include relatively inexpensive parts when compared with the display module.

The display module may include a display module housing, and the sensor module may include a sensor module housing for enclosing the sensor. The modules may include exposed electrical interfaces which connect when the housings are fastened together. The sensor module housing may be translated relative to the display module housing when fastening the housings together to connect the electrical interfaces which are, in turn, no longer exposed. The RBT unit may define a continuous body upon fastening of the housings together.

The display module housing may define a docking bay in which the sensor module housing can be slid, the sensor module housing defining a pair of protruding rails for being received within a pair of elongate recesses defined by the display module housing. The sensor module housing may define a threaded fastening hole which can be placed in register with a fastening hole of the display module housing and receive a fastener to fasten the housings together.

The sensor may include an electro-chemical fuel cell sensor. The electrical interfaces may be power and communications interfaces.

The display module may include:
a display module controller circuitry located within the display module housing and for controlling the display module;
a liquid crystal display (LCD) coupled to the display module controller circuitry and for displaying the indication of BAC in the form of a numeric reading; and
a user interface coupled to the display module controller circuitry and for enabling a user to operate the RBT unit.

The display module controller circuitry may include:
a processor arrangement for executing software;
an exposed display module electrical interface coupled to the processor arrangement and for facilitating powering of and communication with the sensor module;
a battery and charging system for powering the processor arrangement and charging an internal battery;
a blue tooth communication transceiver module for interfacing with external devices. and
a global positioning system module for sensing the position of the RBT unit to be stored by the processor arrangement in use.

The sensor module may include:
a processor arrangement for executing software;
an exposed sensor module electrical interface coupled to the processor arrangement and for facilitating receipt of power from and communication with the display module;
an electro-chemical fuel cell sensor for sensing BAC and coupled to the processor arrangement; and
a backup battery for providing auxiliary power to the processor arrangement.

The sensor module processor arrangement may include non-volatile memory for storing a unique identifier in the form of a serial number.

The sensor module may further include a pressure sensor for measuring the internal pressure of the sensor module. The sensor module may further include a pump and solenoid.

According to another aspect of the present disclosure, there is provided a replaceable sensor module for a hand-held random breath test (RBT) unit, the sensor module configured to be releasably fastened to a display module for displaying an indication of blood alcohol content (BAC), the sensor module enclosing a sensor for sensing the BAC.

According to another aspect of the present disclosure, there is provided a display module for a hand-held random breath test (RBT) unit, the display module configured to display an indication of blood alcohol content (BAC) and able to be releasably fastened with a sensor module, the sensor module enclosing a sensor for sensing the BAC.

According to another aspect of the present disclosure, there is provided a method of assembling a hand-held random breath test (RBT) unit, the RBT unit including a display module for displaying an indication of blood alcohol content (BAC) and a sensor module enclosing a sensor for sensing the BAC, the method including the step of:
releasably fastening the sensor module relative to the display module.

The step of releasably fastening may involve translating (or sliding) the sensor module relative to the display module. The sensor may be translated so that exposed electrical interfaces of the modules connect and are, in turn, no longer exposed.

The step of fastening may further include the step of fastening the sensor module to the display module with a threaded fastener.

The method may further include the step of actuating a user interface of the display module.

Responsive to actuating a user interface, the method may further include the steps of:
verifying calibration settings of the sensor module with the display module; and
storing a calibration record forming a traceable record which can be used in the event of a legal challenge to the integrity of RBT unit.

The step of verifying may involve comparing calibration settings of the sensor module with predetermined acceptable limits. The step of verifying may involve: the display module determining whether a unique identifier stored in the sensor module is valid; the display module determining whether a model number stored in the sensor module is valid; and the display module determining whether the firmware of the sensor module is valid.

The calibration record may be stored in the display module and include: unique serial numbers of both the display module and sensor module; firmware version numbers of both the display module and sensor module; the date and time of actuating the user interface, and verification confirmation of calibration settings of the sensor module by the display module.

The method may further include the step of the display module polling to determine when the sensor module is unfastened from the display module.

The method may further include the steps of:
monitoring the time elapsed relative to a last calibration date of the sensor module; and
issuing notifications both immediately prior to and upon the time elapsed exceeding a predetermined non-calibration period.

The method may further include the steps of:
tracking the date and time independently in each module; and
responsive to detecting a difference in the tracked dates or times, setting the date and time of the display module to that of the sensor module.

The method may further include the step of replacing the sensor module with another sensor module during calibration of the sensor module. The step of replacing may involve unfastening the threaded fastener and translating (or sliding) the sensor module from the display module.

According to another aspect of the present disclosure, there is provided a method of disassembling a hand-held random breath test (RBT) unit, the RBT unit including a display module for displaying an indication of blood alcohol content (BAC) and a sensor module enclosing a sensor for sensing the BAC, the method including the step of:
unfastening the sensor module relative to the display module.

Any of the features described herein can be combined in any combination with any one or more of the other features described herein within the scope of the disclosure.

### BRIEF DESCRIPTION OF THE DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of the Invention in any way. The Detailed Description will make reference to a number of drawings as follows:
Figure 1 is a perspective view of a hand-held random breath test (RBT) unit in accordance with an embodiment of the present invention;
Figures 2a ― 2c show a sequence of steps associated with a method of assembling the hand-held RBT unit of Figure 1;
Figure 3 is a block diagram of the hand-held RBT unit of Figure 1; and
Figure 4 is a flowchart of a method of assembling the hand-held RBT unit of Figure 1; and
Figure 5 is a front view of the unit of Figure 1 with a drug sample cartridge.

### DETAILED DESCRIPTION OF PREFERRED EMBODIMENTS

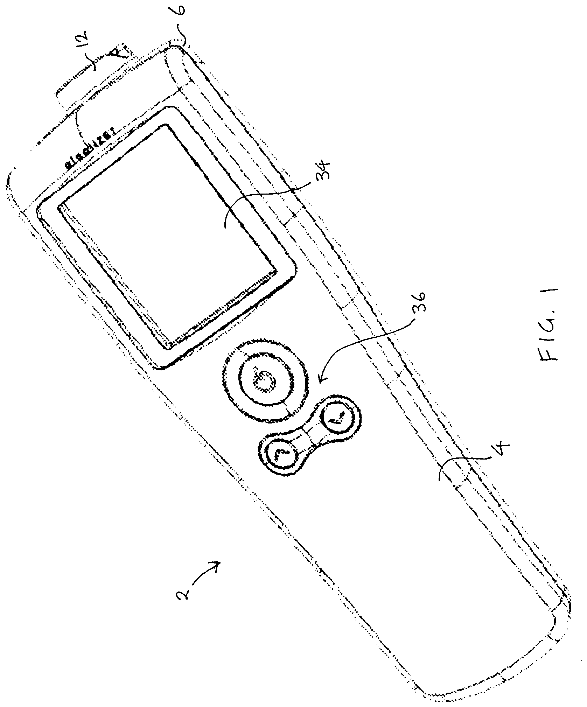

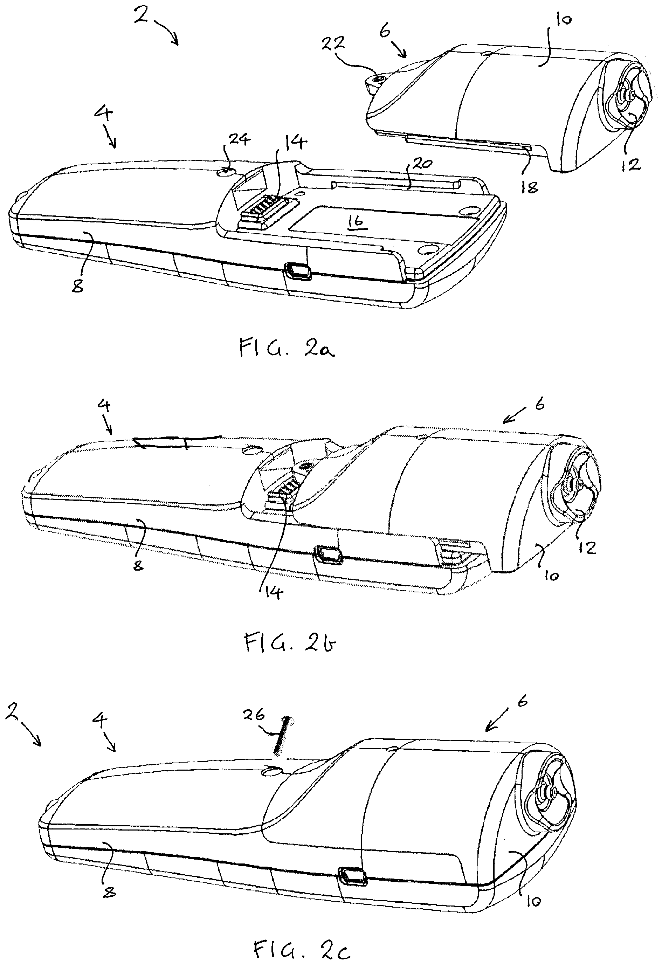

According to an embodiment of the present invention, there is provided a hand-held random breath test (RBT) unit 2 as shown in Figure 1. As can best be seen in Figure 2, the modular RBT unit 2 includes a display module 4 for displaying an indication of blood alcohol content (BAC) and a sensor module 6 for releasably fastening to the display module 4. The sensor module 6 includes a BAC sensor for sensing the BAC and can be released from the display module 4 for calibration. The exposed sensor module 6 houses the BAC sensor so that damage to the sensor or the compromising of its calibration is impeded. Accordingly, the integrity of the RBT unit 2 is improved when compared with other RBT units having exposed releasable BAC sensors.

The BAC sensor is completely contained or encapsulated within the sensor module 6 and cannot be accessed with a finger. The BAC sensor is also fluidically sealed in an air-tight and water-tight manner within the sensor module 6 to impede exposure of the sensor to the elements when the RBT unit 2 is used in the rain or dropped in a puddle, for example. The sensor module 6 can be either reusable, or disposable whereby it includes relatively inexpensive parts when compared with the display module 4. A detailed description of the RBT unit 2 is provided below.

Turning to Figure 2, the display module 4 includes a display module housing 8, and the sensor module 6 includes a sensor module housing 10 for housing the sensor. The sensor module housing 10 has a removable mouthpiece attachment 12 with inlet through which a breath sample can be provided. The modules 4, 6 include exposed electrical interfaces 14 which connect when the housings 4,6 are fastened together. With reference to Figure 2b, the sensor module housing 10 is translated relative to the display module housing 8 when fastening the housings 8, 10 together to connect the electrical interfaces 14. Upon fastening the housings 8, 10 together, the electrical interfaces 14 are no longer exposed and the RBT unit 2 defines a continuous body as shown in Figure 2c.

With reference to Figure 2a, the display module housing 8 defines a docking bay 16 in which the sensor module housing 10 can be slid. The sensor module housing 10 defines a pair of opposed protruding rails 18 for being received within a pair of elongate recesses 20 defined by the display module housing 8. The sensor module housing 10 also defines a threaded fastening hole 22 which can be placed in register with a fastening hole 24 of the display module housing 8. As shown in Figure 2c, the holes 22, 24 can receive a threaded screw 26 (i.e. fastener) to fasten the housings 8, 10 together.

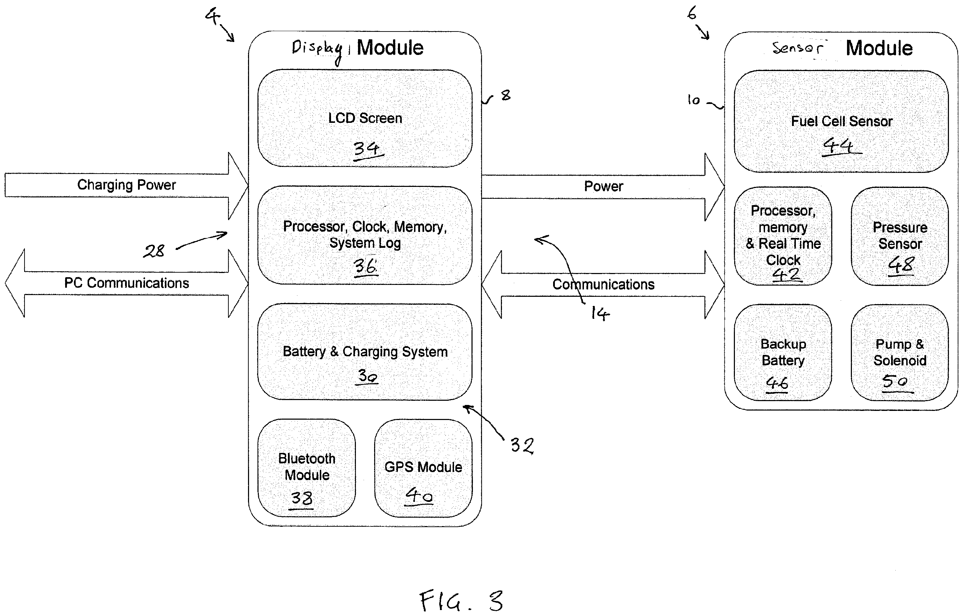

Turning to Figure 3, the electrical interfaces 14 of the display module 4 and sensor module 6 are power and communications interfaces. In addition, the display module 4 further includes another electrical interface 28 through which power is supplied to a battery and charging system 30 and for communications with a personal computer (PC).

The display module 4 includes display module controller circuitry 32 within the display module housing 8 and that is used to control the display module 4. A liquid crystal display (LCD) 34 is coupled to the display module controller circuitry 32 and displays the indication of BAC in the form of a numeric reading. Turning briefly to Figure 1, a user interface 36 is also coupled to the display module controller circuitry 32 and enables a user to operate the RBT unit 2.

Returning to Figure 3, the display module controller circuitry 32 includes a processor arrangement 36 for executing software in the form of computer readable instructions to operate the display module 4. The display module controller circuitry 32 also includes the exposed display module electrical interface 14 coupled to the processor arrangement 36 and for facilitating powering of and communication with the sensor module 6. The internal battery and charging system 30 is suitable for powering the processor arrangement 36 and charging an internal battery using power supplied through the interface 28. A blue tooth communication transceiver module 38 can be used to download a calibration record stored by the processor arrangement 36 from the RBT unit 2 and can interface with external devices. The display module controller circuitry 32 also includes a global positioning system module 40 for sensing the position of the RBT unit 2 to be stored by the processor arrangement 36 during testing.

The sensor module 6 includes a processor arrangement 42 for executing software in the form of computer readable instructions to operate the sensor module 6. The processor arrangement 42 includes non-volatile memory for storing a unique identifier in the form of a serial number corresponding to the sensor module 6. Further, the sensor module 6 includes the exposed sensor module electrical interface 14 coupled to the processor arrangement 42 and for facilitating receipt of power from and communication with the display module 4. The internal BAC sensor 44 is an electro-chemical fuel cell sensor for sensing BAC and is coupled to the processor arrangement 42. The sensor module 6 further includes a backup battery 46 for providing auxiliary power to the processor arrangement 42.

The sensor module 6 further includes known components to facilitate the breath test. In particular, the sensor module 6 includes a pressure sensor 48 for measuring the breath volume delivered to the sensor module 6 during the test and during calibration of the 44. The sensor module 6 further includes a pump and solenoid 50 coupled to the mouthpiece attachment point and inlet 12 through which the breath sample is provided during testing.

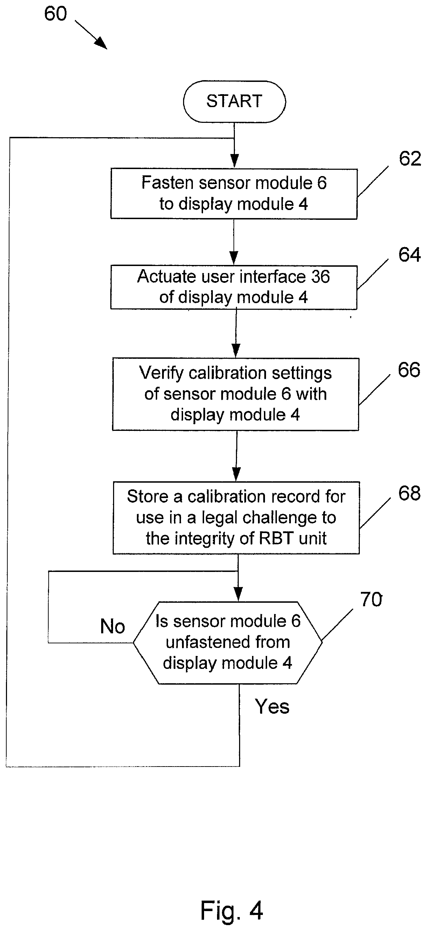

A method 60 of assembling the hand-held random breath test (RBT) unit 2 will now be described with reference to Figure 4.

Initially, the display module 4 and the sensor module 6 are separated as shown in Figure 2a.

At step 62, the sensor module 6 is releasably fastened relative to the display module 4. Elaborating further, the sensor module 6 is translated or slid relative to the display module 4 as shown in Figure 2b. The sensor module 6 is translated so that exposed electrical interfaces 14 of the modules 4, 6 connect and are, in turn, no longer exposed as shown in Figure 2c. The screw 26 is then received by the modules 4, 6 to impede their separation.

At step 64, the user interface 36 of the display module 4 is actuated by pressing any actuator.

At step 66 and responsive to actuating the user interface 36 at step 64, calibration settings of the sensor module 6 are verified with the display module 4. This step of verifying involves comparing stored calibration settings in the processor arrangement 42 of the sensor module 6 with predetermined acceptable limits stored in the processor arrangement 36 of the display module 4. This step of verifying also involves: the display module 4 determining whether the unique identifier stored in the processor arrangement 42 of sensor module 6 is valid; the display module 4 determining whether a model number stored in the processor arrangement 42 of sensor module 6 is valid; and the display module 4 determining whether the firmware of the sensor module 6 is valid.

At step 68, a calibration record is stored that forms a traceable record which can be used in the event of a legal challenge to the integrity of RBT unit 2. The calibration record is stored in the processor arrangement 36 of the display module 4 and includes: unique serial numbers of both the display module 4 and sensor module 6; firmware version numbers of both the display module 4 and sensor module 6; the date and time of actuating the user interface 36 in step 64, and verification confirmation of calibration settings of the sensor module 6 by the display module 4 performed at step 66.

At query step 70, the display module 4 polls the user interface 14 to determine when the sensor module 6 is unfastened from the display module 4. If the sensor module 6 is not unfastened from the display module 4, the method 60 remains at step 70 and the RBT unit 2 can operate normally to perform RBTs.

If the sensor module 6 is unfastened from the display module 4, the method 60 returns to step 62 where the sensor module 6 can be replaced with another sensor module during offsite calibration of the sensor module 6. During this procedure, the threaded fastener 26 is removed and the sensor module 6 is slid from the display module 8 to unfasten the sensor module 6 from the display module 4.

In conjunction with the method 60 described above, the RBT unit 2 also performs the background steps of:
monitoring the time elapsed relative to a last calibration date stored in the sensor module; and
issuing warning notifications to the user on the LCD 34 both immediately prior to and upon the time elapsed exceeding a predetermined non-calibration period.

In conjunction with the method 60 described above, the RBT unit 2 also performs the background steps of:
tracking the date and time independently in each module 4, 6; and
responsive to detecting a difference in the tracked dates or times, setting the date and time of the display module 4 to that of the sensor module 6.

The assembly and disassembly of the RBT unit 2 described above in relation to method 60 is straightforward and can be readily conducted by police officers without the need for any formal training. In addition, the method 60 can be conducted in poor weather conditions without fear of damaging or affecting calibration of the internal BAC sensor 44.

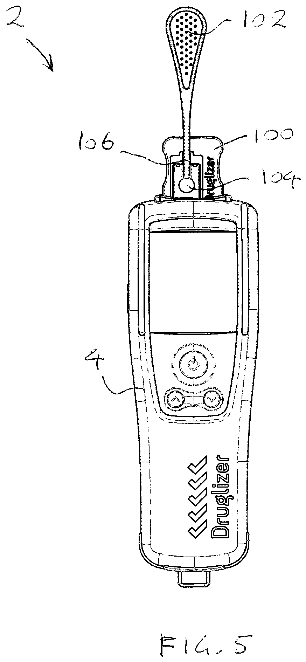

Turning to Figure 5, the hand-held RBT unit 2 is a mind-altering substance testing unit 2 for testing for a drug as well as alcohol. In particular, the mouthpiece 12 of the sensor module 6 can be removed and a drug sample cartridge 100 docked to the sensor module 6 in its place. Advantageously, the same testing unit 2 can be used to sense alcohol and the drug avoiding the need for separate testing units.

The sample cartridge 100 receives a saliva sample and is releasably fastened to the sensor module 6 so that the sensor module 6 can sense the mind-altering drug. The unit 2 further includes a swab 102 including the saliva sample. The swab 102 is fastened to the cartridge 100 by inserting a sample end 104 in a cartridge sample dock 106.

The sensor module 6 includes a lateral flow sensor for sensing the drug and wholly enclosed within the sensor module 6. The single-use cartridge 100 is disposable, yet includes an onboard electronic memory for storing information. The stored information relates to the age of the cartridge 100 or batch parameters of the cartridge 100. The sensor module 6 compensates sensing in accordance with the stored information for improved accuracy.

The display module 4 and sensor module 6 drug functionality is the same as that of the alcohol functionality previously described.

A person skilled in the art will appreciate that many embodiments and variations can be made without departing from the ambit of the present invention.

In compliance with the statute, the invention has been described in language more or less specific to structural or methodical features. It is to be understood that the invention is not limited to specific features shown or described since the means herein described comprises preferred forms of putting the invention into effect.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment. Furthermore, the particular features, structures, or characteristics may be combined in any suitable manner in one or more combinations.

## Claims

1. A mind-altering substance testing unit (2) including a display module (4) for displaying an indication of at least one mind-altering substance; **characterized in that** the test unit (2) further includes:
a sensor module (6) for releasably fastening to the display module (4) and including a sensor for sensing the at least one mind-altering substance; and
a sample cartridge (100) for receiving a sample, the sample being a saliva sample, and for releasably fastening to the sensor module (6) in use so that the sensor module (6) can sense the at least one mind-altering substance.

2. A mind-altering substance testing unit (2) as claimed in claim 1, further including a swab (102) including the sample and for fastening to the cartridge (100).

3. A mind-altering substance testing unit (2) as claimed in claim 1 or claim 2, wherein the at least one mind-altering substance is a drug, and the sensor module (6) includes a lateral flow sensor.

4. A mind-altering substance testing unit (2) as claimed in claim 1 or claim 2, wherein the at least one mind-altering substance is alcohol and the sensor module (6) includes an electro-chemical fuel cell sensor blood alcohol content (BAC).

5. A mind-altering substance testing unit (2) as claimed in claim 1 or claim 2, wherein the at least one mind altering substance includes a drug and alcohol, and the sensor module (6) includes a lateral flow sensor for sensing the drug and an electro-chemical fuel cell for sensing the alcohol.

6. A mind-altering substance testing unit (2) as claimed in any one of the preceding claims, wherein the sample cartridge (100) is disposable.

7. A mind-altering substance testing unit (2) as claimed in any one of the preceding claims, wherein the sample cartridge (100) includes an electronic memory for storing information.

8. A mind-altering substance testing unit (2) as claimed in claim 7, wherein the information relates to the age of the sample cartridge (100) or batch parameters of the sample cartridge (100).

9. A mind-altering substance testing unit (2) as claimed in claim 7 or claim 8, wherein the sensor module (6) compensates sensing in accordance with the stored information for improved accuracy.

10. A mind-altering substance testing unit (2) as claimed in any one of the preceding claims, wherein the sensor module (6) includes a mouthpiece (12) for releasably fastening to the sensor module (6) in place of the sample cartridge (100).

11. A mind-altering substance testing unit (2) as claimed in any one of the preceding claims, wherein the unit (2) is hand-held.

12. A mind-altering substance testing unit (2) as claimed in any one of the preceding claims, wherein the sensor is wholly enclosed within the sensor module (6).

## Patentansprüche

1. Testeinheit (2) für eine bewusstseinsverändernde Substanz, die ein Anzeigemodul (4) zum Anzeigen einer Angabe mindestens einer bewusstseinsverändernden Substanz einschließt; **dadurch gekennzeichnet, dass** die Testeinheit (2) weiter Folgendes einschließt:
ein Sensormodul (6) zum lösbaren Befestigen an dem Anzeigemodul (4) und einschließend einen Sensor zum Messen der mindestens einen bewusstseinsverändernden Substanz; und
eine Probenkartusche (100) zum Aufnehmen einer Probe, wobei die Probe eine Speichelprobe ist, und zum lösbaren Befestigen an dem in Gebrauch befindlichen Sensormodul (6), sodass das Sensormodul (6) die mindestens eine bewusstseinsverändernde Substanz messen kann.

2. Testeinheit (2) für eine bewusstseinsverändernde Substanz nach Anspruch 1, die weiter einen Abstrichtupfer (102), einschließend die Probe und zum Befestigen an der Kartusche (100), einschließt.

3. Testeinheit (2) für eine bewusstseinsverändernde Substanz nach Anspruch 1 oder Anspruch 2, wobei die mindestens eine bewusstseinsverändernde Substanz eine Droge ist und das Sensormodul (6) einen Lateral-Flow-Sensor einschließt.

4. Testeinheit (2) für eine bewusstseinsverändernde Substanz nach Anspruch 1 oder Anspruch 2, wobei die mindestens eine bewusstseinsverändernde Substanz Alkohol ist und das Sensormodul (6) eine elektrochemische Brennstoffzelle als Sensor zum Messen des Blutalkoholgehalts (blood alcohol content, BAC) einschließt.

5. Testeinheit (2) für eine bewusstseinsverändernde Substanz nach Anspruch 1 oder Anspruch 2, wobei die mindestens eine bewusstseinsverändernde Substanz eine Droge und Alkohol einschließt und das Sensormodul (6) einen Lateral-Flow-Sensor zum Messen der Droge und eine elektrochemische Brennstoffzelle zum Messen des Alkohols einschließt.

6. Testeinheit (2) für eine bewusstseinsverändernde Substanz nach einem der vorstehenden Ansprüche, wobei die Probenkartusche (100) eine Einweg-Probenkartusche ist.

7. Testeinheit (2) für eine bewusstseinsverändernde Substanz nach einem der vorstehenden Ansprüche, wobei die Probenkartusche (100) einen elektronischen Speicher zum Speichern von Informationen einschließt.

8. Testeinheit (2) für eine bewusstseinsverändernde Substanz nach Anspruch 7, wobei sich die Informationen auf das Alter der Probenkartusche (100) oder die Chargenparameter der Probenkartusche (100) beziehen.

9. Testeinheit (2) für eine bewusstseinsverändernde Substanz nach Anspruch 7 oder Anspruch 8, wobei das Sensormodul (6) das Messen in Übereinstimmung mit den gespeicherten Information für eine verbesserte Genauigkeit ausgleicht.

10. Testeinheit (2) für eine bewusstseinsverändernde Substanz nach einem der vorstehenden Ansprüche, wobei das Sensormodul (6) ein Mundstück (12) zum lösbaren Befestigen an dem Sensormodul (6) anstelle der Probenkartusche (100) einschließt.

11. Testeinheit (2) für eine bewusstseinsverändernde Substanz nach einem der vorstehenden Ansprüche, wobei die Einheit (2) tragbar ist.

12. Testeinheit (2) für eine bewusstseinsverändernde Substanz nach einem der vorstehenden Ansprüche, wobei der Sensor vollständig von dem Sensormodul (6) umschlossen ist.

## Revendications

1. Unité de test de substance altérant l'esprit (2) incluant un module d'affichage (4) pour afficher une indication d'au moins une substance altérant l'esprit ; **caractérisée en ce que** l'unité de test (2) inclut en outre :
un module de capteur (6) destiné à être fixé de manière amovible au module d'affichage (4) et incluant un capteur pour détecter l'au moins une substance altérant l'esprit ; et
une cartouche d'échantillon (100) destinée à recevoir un échantillon, l'échantillon étant un échantillon de salive, et pour être fixée de manière amovible au module de capteur (6) en utilisation de telle sorte que le module de capteur (6) puisse détecter l'au moins une substance altérant l'esprit.

2. Unité de test de substance altérant l'esprit (2) selon la revendication 1, incluant en outre un écouvillon (102) incluant l'échantillon et destiné à être fixé à la cartouche (100).

3. Unité de test de substance altérant l'esprit (2) selon la revendication 1 ou la revendication 2, dans laquelle l'au moins une substance altérant l'esprit est un médicament et le module de capteur (6) inclut un capteur d'écoulement latéral.

4. Unité de test de substance altérant l'esprit (2) selon la revendication 1 ou la revendication 2, dans laquelle l'au moins une substance altérant l'esprit est un alcool et le module de capteur (6) inclut un taux d'alcool dans le sang (BAC) de capteur de pile à combustible électrochimique.

5. Unité de test de substance altérant l'esprit (2) selon la revendication 1 ou la revendication 2, dans laquelle l'au moins une substance altérant l'esprit inclut un médicament et un alcool et le module de capteur (6) inclut un capteur d'écoulement latéral pour détecter le médicament et une pile à combustible électrochimique pour détecter l'alcool.

6. Unité de test de substance altérant l'esprit (2) selon l'une quelconque des revendications précédentes, dans laquelle la cartouche d'échantillon (100) est jetable.

7. Unité de test de substance altérant l'esprit (2) selon l'une quelconque des revendications précédentes, dans laquelle la cartouche d'échantillon (100) inclut une mémoire électronique pour stocker des informations.

8. Unité de test de substance altérant l'esprit (2) selon la revendication 7, dans laquelle les informations se rapportent à l'âge de la cartouche d'échantillon (100) ou de paramètres de lot de la cartouche d'échantillon (100).

9. Unité de test de substance altérant l'esprit (2) selon la revendication 7 ou la revendication 8, dans laquelle le module de capteur (6) compense la détection selon les informations stockées pour une meilleure précision.

10. Unité de test de substance altérant l'esprit (2) selon l'une quelconque des revendications précédentes, dans laquelle le module de capteur (6) inclut un embout buccal (12) destiné à être fixé de manière amovible au module de capteur (6) à la place de la cartouche d'échantillon (100).

11. Unité de test de substance altérant l'esprit (2) selon l'une quelconque des revendications précédentes, dans laquelle l'unité (2) est portative.

12. Unité de test de substance altérant l'esprit (2) selon l'une quelconque des revendications précédentes, dans laquelle le capteur est complètement enfermé à l'intérieur du module de capteur (6).
